# EUROPEAN PATENT SPECIFICATION

(11) **EP 0 383 914 B1**
(45) Date of publication and mention of the grant of the patent: **14.12.1994**
(21) Application number: 88908395.2
(22) Date of filing: 01.10.1988
(51) Int. Cl.: A61M 25/00

(54) **CATHETER**
KATHETER
CATHETER

(30) Priority: 02.10.1987 JP 248101/87; 05.10.1987 JP 249911/87; 22.03.1988 JP 65831/88; 13.05.1988 JP 114902/88
(43) Date of publication of application: 29.08.1990
(73) Proprietor: TERUMO KABUSHIKI KAISHA, Tokyo 151 (JP); HIROSE, Shigeo, Tokyo 152 (JP)
(72) Inventor: HIROSE, Shigeo, Meguro-ku Tokyo 152 (JP); HARADA, Fumiaki, Fuji-shi Shizuoka 417 (JP); ISHIDA, Toshinobu, Fuji-shi Shizuoka 417 (JP)
(74) Representative: Casalonga, Axel
(86) International application number: JP8801010
(87) International publication number: WO8902762

(56) References cited:
- EP-A- 0 199 870
- DE-A- 3 707 899
- JP-U- 592 344
- PATENT ABSTRACTS OF JAPAN, vol. 8, no. 151 (P-286)[1588], 13th July 1984;& JP-A-59 48 710 (SUMITOMO DENKI KOGYO K.K.) 21-03-1984

## Description

The present invention relates to a catheter which is inserted for use into a tubular organ of an organism, such as a blood vessel, an alimentary canal or an air tube.

Generally, the selective angiography or angiectasia employs catheters or guiding catheters of various shapes and dimensions. In either of these techniques, an optimal catheter must be selected from among various types of catheter. It often occurs that no optimal catheter is found in the ready-made ones. Also, insertion of the catheter to an objective position in a blood vessel which extends or branches out in a complicated manner requires a complicated operation such as twisting and a high skill.

Accordingly, a catheter which exhibits excellent operability when placed in a tubular organ is disclosed in , for example, Japanese Patent Laid-Open Nos. 131758/1986 and 255669/1986. In such catheters, a shape memory alloy is incorporated in a catheter. The shape memory alloy is deformed by means of the heating, by which the catheter is bent in a desired manner.

EP-A-0 199 870 discloses a catheter, which is to be inserted into a tubular organ, with at least two unidirectional shape memory alloy wire members, which recover their original shape at a temperature higher than a set temperature and which can undergo deformation at a temperature lower than the set temperature, provided at least in the distal end portion of said catheter substantially parallel to an axis of said catheter and at positions which ensure that the force generated when at least one of said unidirectional shape memory alloy wire members is heated to the temperature higher than the set temperature and thereby recovers its original shape exerts on the other unidirectional shape alloy wire member(s), having a temperature lower than the set temperature, a bending force and a cylindrical supporting member being provided inside of said unidirectional shape memory alloy wire members.

However, the above-described conventional catheters utilize changes in the curvature of or in the complicated shape of the shape memory alloy and therefore require a very large amount of force of the shape memory alloy. However, the force with which the shape memory alloy deforms into a complicated shape, e.g., it is bent, is not large, and this necessitates provision of a shape memory alloy which is large in size.

An object of the present invention is to provide a catheter which has a simple structure, which is capable of being bent in a desired form and which exhibits excellent operability when it is inserted into a tubular organ.

According to a first aspect of the invention, a catheter to be inserted into a tubular organ comprises at least two unidirectional shape memory alloy wire members, which recover their original shape at a temperature higher than a set temperature and which undergo deformation at a temperature lower than the set temperature, provided at least in the distal end portion of the catheter substantially parallel to an axis of said catheter and at positions which ensure that the force generated when at least one of the unidirectional shape memory alloy wire members is heated to the temperature higher than the set temperature and thereby recovers its original shape exerts on the other unidirectional shape memory alloy wire member having the temperature lower than the set temperature a bending force, and a cylindrical supporting member provided inside of said unidirectional shape memory alloy wire members, characterized in that said supporting member is adapted to be easily flexed but no easily compressed in its longitudinal direction, and exerts a straightening recovering force to the shape memory alloy wire members of the distal end portion when said supporting member recovers its initial shape.

Another feature of the present invention is that the distal end portion of the catheter is made of a material which is relatively flexible, and the portion of the catheter other than the distal end portion thereof is made of a material which is more rigid than that of the distal end portion.

Another feature of the present invention is that the unidirectional shape memory alloy wire members are provided along both the distal end portion and the portion of the catheter other than the distal end portion, one end of each of the unidirectinal shape memory alloy wire members being fixed to the distal end portion and the other end thereof being fixed to the portion other than the distal end portion.

Another feature of the present invention is that the supporting member is a tight spring.

Another feature of the present invention is that two unidirectional shape memory alloy wire members are disposed at positions which are opposed each other with respect to a central axis of the catheter.

Another feature of the present invention is that three or more unidirectional shape memory alloy wire members are disposed at positions which form a polygon within which a central axis of the catheter is disposed.

Another feature of the present invention is that the unidirectional shape memory alloy wire members are provided in a state in which they are elongated in their longitudinal direction.

Another feature of the present invention is that the unidirectional shape memory alloy wire members are heated by means of the heat generated by an internal resistance thereof when energized.

In the catheter according to the present invention, the shape memory alloy wire members (A, B...) are arranged such that they recover their original shape and are straight at a temperature equal to or higher than the temperature of an organism (at the inverse transformation temperature or above) and such that it is deformed and therefore elongated in their longitudinal direction at the temperature which is in the vicinity of the temperature of an organism (at the transformation temperature or below). When one (A) of the shape memory alloy wire members which are in an elongated state is, for example, energized and thereby heated to the inverse transformation srarting temperature or above by means of the heat generated due to an internal resistance thereof as a consequence of energization, the shape memory alloy wire member (A) recovers its original shape, i.e., contraction, and becomes straight. At that time, the contracting force of the shape memory alloy wire member (A) acts on the other shape memory allory wire member (B ...) exerting a bending force, and the distal end portion of the catheter is therefore curved at a fixed curvature in a state where the side of the distal end portion in which the shape memory alloy wire member (A) is provided is concave. Thereafter, the other shape memory alloy wire member (B ...) is heated to the inverse transformation temperature or above by means of the heat generated due to its internal resistance when it is, for example, energized. In consequence, the shape memory alloy wire member (B ...) recovers its original shape, i.e., becomes straight and contracted. As a result, all the shape memory alloy wire members (A, B ...) recover their original shape, and the distal end portion of the catheter is thereby made straight.

The curved state (the curvature and the direction of the curve) of the distal end portion of the catheter can be freely selected by controlling the degree at which the shape memory alloy wire members (A, B...) recover thier original shape.

In the case of the catheter according to the present invention in which the distal end portion is formed of a material which is relatively flexible and the portion of the catheter other than the distal end portion is made of a material which is more rigid than that of the distal end portion, even if the deforming force of all the shape memory alloy wire members is small, the distal end portion of the catheter can be curved adequately. Also, the use of thin and small shape memory alloy wire members is enabled.

In the case of the catheter according to the present invention in which the shape memory alloy wire members are provided along both of the distal end portion and the portion other than the distal end portion (e.g., along the substantially overall length of the catheter), the length of the shape memory alloy wire members provided in the catheter is long. In consequence, the degree of extension or contraction (the deformed amount) of the shape memory alloy wire members increases, and this allows the distal end portion of the catheter to be curved at a large curvature. Also, the use of thin and short shape memory alloy wire members is enabled. At that time, if the distal end portion of the catheter is made of a material which is softer than that of the portion other than the distal end portion, the large degree of extension or contraction of the shape memory alloy wire members can be concentrated on the soft portion which forms the forward end of the catheter, allowing it to be curved more freely at a larger curvature. Also, the use of thin and short alloy wire members is enabled.

In the case of the catheter according to the present invention in which a cylindrical supporting member, which is flexible in a direction in which it is bent and which is not easily compressed in its longitudinal direction, is provided inside of the shape memory alloy wire members (A, B...), when one shape memory alloy wire member (A) returns to its original state and is contracted, the contracting force of the shape memory alloy wire member (A) is exerted only as the bending force which bends the distal end portion of the catheter due to the presence of the cylindrical supporting member. As a result, deformation of the shape memory alloy wire member (A) can be effectively utilized to bend the catheter.

The unidirectional shape memory alloy employed in the catheter according to the present invention is of the type which generates thermo-elastic martensite transformation and recovers its original shape when heated to the inverse transformation starting temperature or above. The shape memory alloy undergoes deformation at the temperature lower than the martensite transformation temperature. Once the shape memory alloy is heated to the inverse transformation stating temperature or above, even if the temperature of the shape memory alloy lowers to the martensite transformation temperature or below, it maintains the original shape as long as no external force is applied thereto.

It is preferable for the unidirectional shape memory alloy employed in the catheter according to the present invention to be made of a Ti-Ni type alloy which contains 51 to 50 percents by atom of Ni and which has an inverse transformation temperature ranging from 40 °C to 100 °C (if the inverse transformation temperature is 40°C or above, the shape memory alloy is not easily affected by the body temperature, and if the inverse transformation temperature is 100 °C or below, the shape memory alloy less affects the body or the material which constitutes the catheter). More preferably, the inverse transformation temperature ranges form 45 °C to 55°C.

According to a second aspect of the present invention, the catheter to be inserted into a tubular organ has at least one flow passage which is formed therethrough along the overall length thereof, at least one bidirectional shape memory alloy wire member provided in a wall on an inner wall surface or on an outer wall surface of the catheter in such a manner that it extends in an axial direction of the catheter, so that the shape of a distal end portion of the catheter reversibly changes by changing the temperature of the wire member, and a cylindrical supporting member provided inside of said wall having the shape memory alloy member and is characterized in that said supporting member is adapted to be easily flexed but not easily compressed and exerts a recovering force to the shape memory alloy wire member of the distal end portion of the catheter when said cylindrical supporting member recovers its initial shape.

Another feature of the present invention is that the distal end portion of the catheter is made of a material which is relatively flexible, and the portion of the catheter other than the distal end portion thereof is made of a material which is more rigid than that of the distal end portion.

Another feature of the present invention is that the bidirectional shape memory alloy wire members are provided along both of the distal end portion and the portion of the catheter other than the distal end portion, one end of each of the bidirectional shape memory alloy wire members being engaged to the distal end portion, and the other end thereof being engaged to the portion other than the distal end portion.

Another feature of the present invention is that the bidirectional shape memory alloy wire member recovers its two original shapes at temperatures which are substantially higher than a body temperature.

Another feature of the present invention is that an axis of the bidirectional shape memory alloy wire member is substantially parallel to an axis of the catheter.

Another feature of the present invention is that the bidirectional shape memory alloy wire member changes its longitudinal shape as the temperature thereof changes.

Another feature of the present invention is that two bidirectional shape memory alloy wire members are disposed at positions which oppose each other with respect to a central axis of the catheter.

Another feature of the present invention is that three or more bidirectional shape memory alloy wire members are disposed at positions which form a polygon within which a central axis of the catheter is disposed.

Another feature of the present invention is that the bidirectional shape memory alloy wire member is heated by means of the heat generated due to its internal resistance then the bidirectional shape memory alloy wire member is energized.

The shape memory alloy wire member (A, B...) which is incorporated in the catheter according to the present invention is arranged such that it reversibly changes its longitudinal shape as the temperature thereof changes, e.g., such that it recovers its original shape and is contracted at the higher temperature and recovers its original shape and is elongated at the lower temperature (which is higher than the body temperature).

In the catheter in which one shape memory alloy wire member (A) is disposed at least in the distal end portion of the catheter parallel to the central axis of the catheter, when the shape memory alloy wire member (A) is heated to the higher temperature by means of the heat generated due to its internal resistance when the shape memory alloy wire member (A) is, for example, energized, it recovers its original shape, i.e., it is contracted in its longitudinal direction. As a result, the shape of the distal end portion of the catheter, which is in its original state, e.g., which is straight, is curved at a fixed curvature in a state in which the side thereof at which the shape memory alloy wire member (A) is provided is concave.

Thereafter, the shape memory alloy wire member (A) is deenergized so that it cools down to the lower temperature. In consequence, the shape memory alloy wire member (A) recovers its original shape and is elongated in its longitudinal direction, causing the shape of the distal end portion of the catheter to return to its initial state, e.g., to its straight state.

In the catheter in which two or more shape memory alloy wire members (A, B...) are disposed apart from each other at least in the distal end portion of the catheter, the distal end portion of the catheter can be curved in an arbitrary direction by selectively deforming arbitrary one of the shape memory alloy members. Furthermore, the distal end portion of the catheter can be curved in a complicated manner by concurrently deforming a plurality of shape memory alloy wire members.

In the curved catheter in which the bidirectional shape memory alloy wire member (A) is provided on the outer side of a curve in the curved catheter, when the bidirectional shape memory alloy wire member (A) is energized and heated to the higher temperature, it recovers its original shape and is contracted, thereby making the catheter straight. When the bidirectional shape memory alloy wire member (A) is deenergized so that it cools down to the lower temperature, it recovers its original shape and is elongated, thereby making the catheter curved again. Deformation of the bidirectional shape memory alloy wire member may be caused not by the change in its longitudinal length but by the change in its curvature.

The curvature at which the distal end portion of the catheter is curved can be freely adjusted by controlling the degree at which the shape memory alloy wire members (A, B...) recover their original shape, which may be achieved by the control of the amount or energization.

The catheter according to the present invention has a simple structure but can be curved at a desired curvature and in a desired direction. It therefore exhibits excellent operability when it is placed within a tubular organ. This enables the catheter according to the present invention to substitute for conventional catheters of various shapes and dimensions and enables the skills required to handle the conventional catheters to be eliminated.

In the catheter according to the present invention in which the distal end portion thereof is made of a material which is relatively flexible and the portion thereof other than the distal end portion is made of a material which is more rigid than that of the distal end portion, even if the deforming force of the shape memory alloy wire member is small, the distal end portion of the catheter can be curved adequately, and the use of thin and short shape memory alloy wire member is enabled.

In the catheter according to the present invention in which the shape memory alloy wire member is provided along both the the distal end portion and the portion other than the distal end portion (e.g., along the substantially overall length of the catheter), the length of the shape memory alloy wire member provided in the catheter is long. In consequence, the degree of extension or contraction (the degree of deformation) of the shape memory alloy wire member increases, and this allows the distal end portion of the catheter to be curved at a large curvature. Also, the use of thin shape memory alloy wire member is enabled. At that time, in a case where the distal end portion of the catheter is made of a material which is softer than that of the portion other than the distal end portion, the large degree of extension or contraction of the shape memory alloy wire member can be concentrated on the soft portion which forms the distal end portion of the catheter, allowing it to be curved more freely at a larger curvature. Also, the use of thin and short alloy wire member is enabled.

The bidirectional shape memory alloy employed in this invention has two transformation points and reversibly recovers its original shapes at the higher temperature and the lower temperature.

It is preferable for the bidirectional shape memory alloy employed in the catheter according to the present invention to be made of a Ti-Ni type alloy (which contains 50 to 53 percents by atom of Ni, more preferably, 50 to 51 percents by atom of Ni, and which has transformation temperatures: Af 50 to 80°C, Mf 40 to 60 °C). The transformation temperature is set within the above-described range for the same reasons as in the case of the unidirectional shape memory alloy.
Fig. 1 is a schematic view of a first embodiment of a catheter according to the present invention;
Fig. 2 is an enlarged cross-sectional view of the essential parts of the catheter of Fig. 1;
Fig. 3 is a section taken along the line III- III of Fig. 2;
Fig. 4 is an electric circuit diagram;
Fig. 5 (A) through 5 (D) illustrate the deformed states of the distal end of the catheter;
Fig. 6 is an enlarged cross-sectional view of the essential parts of a second embodiment of the catheter according to the present invention;
Fig. 7 is a section of the catheter, shoving how three unidirectional shape memory alloy wire members are disposed;
Fig. 8 is a schematic view of a fourth embodiment of the catheter according to the present invention;
Fig. 9 illustrates how the catheter is used;
Fig. 10 shows the cross-section of the catheter;
Fig. 11 is a cross sectional view of the essential parts of a fifth embodiment of the catheter according to the present invention;
Fig. 12 is a schematic view of a sixth embodiment of the catheter according to the present invention;
Fig. 13 is an enlarged cross-sectional view of the essential parts of the catheter of Fig. 12;
Fig. 14 is a section taken along the line XIII- XIII of Fig. 13;
Fig. 15 is an electric circuit diagram;
Fig. 16 (A) through 16 (C) illustrate the deformed states of the distal end of the catheter;
Fig. 17 (A) is a schematic view of a seventh embodiment of the catheter according to the present invention;
Fig. 17 (B) and 17 (C) illustrate the deformed states of the catheter;
Fig. 18 is a schematic view of an eighth embodiment of the catheter according to the present invention;
Fig. 19 illustrates how the catheter is used; and
Fig. 20 shows the cross-section of the catheter.

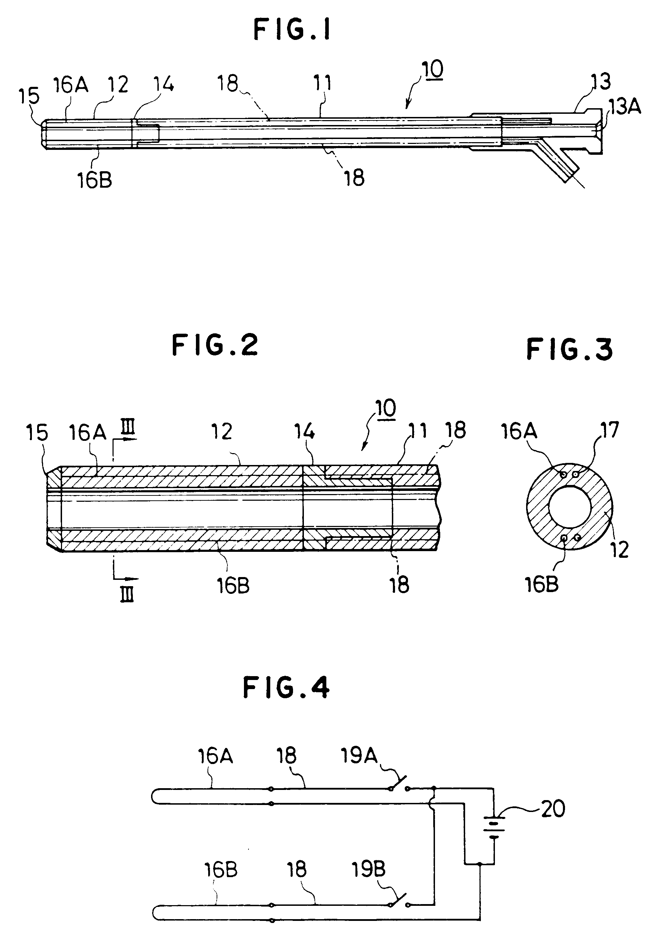

According to a first embodiment, illustrated in figures 1 to 5, a catheter 10 includes a relatively rigid, hollow shaft portion 11, a relatively flexible, hollow distal end portion 12, a hollow hub 13 which is bonded to the proximal end of the shaft portion 11, a relatively rigid, hollow fixed portion 14 which is provided between the shaft portion 11 and the distal portion 12, and a relatively soft, ring-shaped chip 15 which is bonded to the distal end of the distal end portion 12. The shaft portion 11 is made of, for example, polyvinyl chloride resin (which is non-rigid polyvinyl chloride resin containing less amont of plasticizing agent than that that which forms the distal end portion 12). The distal end portion 12 is made of, for example, non-rigid vinyl chloride resin. The hub 13 is made of, for example, polycarbonate. The fixed portion 14 and the soft chip 15 are made of, for example, polyvinyl chloride resin.

The catheter 10 further includes two unidirectional shape memory alloy wire members 16A and 16B which are provided in the distal end portion 12. The two shape memory alloy wire members 16A and 16B are provided on the circumference of the distal end portion 12 at positions which are symmetrical with respect to the central axis of the catheter 10 in such a manner that the longitudial axis thereof extend parallel to the axis of the catheter 10. The shape memory alloy wire members 16A and 16B are inserted into small holes 17 formed through the distal end portion 12. The distal end of each the shape memory alloy wire members 16A and 16B is turned in a U-shaped form, and the proximal end thereof is supported by the fixed portion 14 and connected to leads 18. The fixed portion 14 prevents the deforming force of the shape memory alloy wire members 16A and 16B from being exerted on the shaft portion 11 and the leads 18. The shape memory alloy wire members 16A and 16B recover their original shape, e.g., are straight, at a temperature higher than that of an organism, i.e., in the vicinity of the inverse transformation starting temperature (e.g., 60 °C ). In the vicinity of the temperature of the organism (e.g., 37°C), the shape memory alloy wire members 16A and 16B are deformable in any form, e.g., can be stretched in its longitudinal direction.

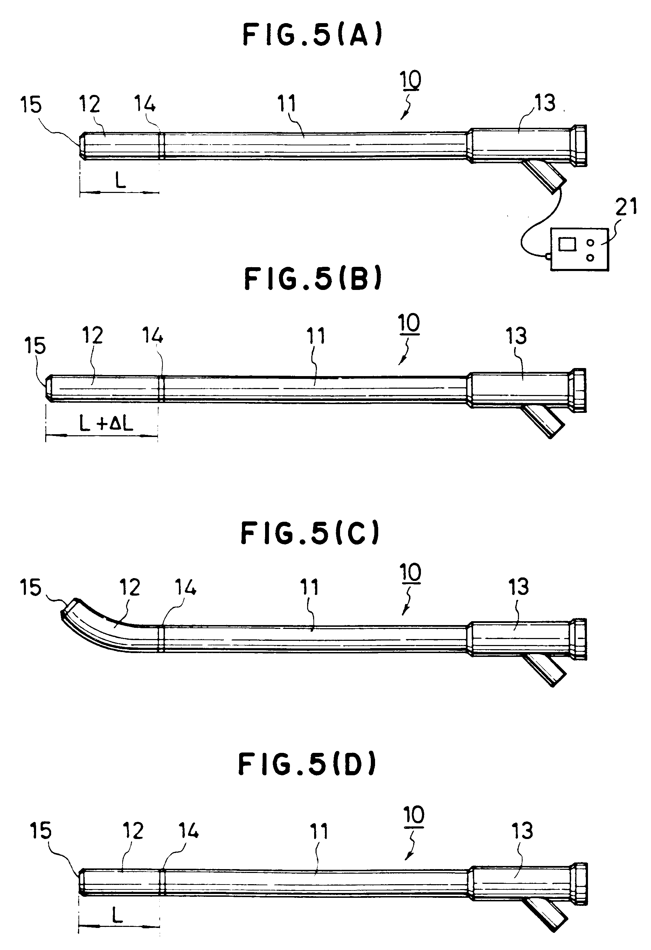

The catheter 10 is arranged such that the force generated when one of the shape memory alloy wire memory 16A (or 16B), which is stretched in its longitudinal direction, is heated to a temperature higher than the temperature of an organism and thereby recovers its original shape exerts on the other shape memory alloy wire member 16B (or 16A) a bending force. The shape memory alloy wire members 16A and 16B are heated by means of the heat generated in the members due to an internal resistance thereof when they are energized by an external power source through the leads 18. Energization of the shape memory alloy wire members is achieved by on/off of switches 19A and 19B. In Fig. 5 (A), a reference numeral 21 denotes a cotrol box which controls heating of the shape memory alloy wire members 16A and 16B.

Next, the operation of the first embodiment will be described.

When the catheter 10 is to be used, the operator forces the distal end portion 12 into an objective position within a blood vessel or the like by manually applying a pushing force to the proximal end portion of the shaft portion 11 while controlling the bending of the catheter distal end portion 12 which occurs when the shape memory alloy wire members 16A and 16B recover their original shape. Thereafter, the operator injects medicine or the like from an injection port 13A of the hub 13.

At that time, the shape memory alloy wire members 16A and 16B in the catheter 10 are employed in a state in which they are stretched by ΔL in their longitudinal direction in the vicinity of the body temperature (see Fig. 5 (B)) from their original length L which is obtained at a temperature (the inverse transformation temperature) which is higher than the temperature of the organism when they are straight (see Fig. 5(A)). When one 16A of the shape memory alloy wire members which are in a stretched state is, for example, energized, and is heated to a temperature equal to or higher than the inverse transformation starting temperature by means of the heat generated due to its internal resistance when the wire member is energized, the shape memory alloy wire member 16A recovers its original shape, i.e., returns to its straight and contracted state. At that time, the contracting force of the shape memory alloy wire member 16A exerts on the shape memory alloy wire member 16B a bending force, and the distal end of the catheter 10 is thereby curved in a state in which the side thereof in which the shape memory alloy wire member 16A is provided is concave (see Fig. 5(C)). Thereafter, the shape memory alloy wire member 16B is heated to a temperature equal to or higher than the inverse transformation starting temperature by means of the heat generated in the shape memory alloy wire member 16B due to its internal resistance when it is, for example, energized, so that the shape memory alloy wire member 16B recovers its original shape, i.e., returns to its contracted straight state. In consequence, all the shape memory alloy wire members 16A and 16B recover their original state, and the distal end of the catheter 10 is contracted to the length L and made straight. (see Fig. 5(D)).

In a case where the shape memory alloy wire members are provided in the distal end portion 12 (having a length L + ΔL) which is made of a resin (e.g., nylon, a fluoroplastic or the like) of the type which easily recovers its original state, in a state where they are stretched to a length L +ΔL (Fig. 5 (B)), when one 16A of the shape memory alloy wire members is heated to a temperature equal to or higher than the inverse transformation starting temperature by means of the heat generated in the shape memory alloy wire member 16A when it is, for example, energized, it recovers its original shape, i.e., it is contracted. At that time, the contracting force of the shape memory alloy wire member 16A exerts on the vicinity of the distal end of the catheter a bending force, and the distal end portion of the catheter 10 is thereby curved at a fixed curvature in a state in which the side thereof in which the shape memory alloy wire member 16A is provided is concave (Fig. 5 (C)). Thereafter, the shape memory alloy wire member 16A is deenergized and is thus extended (to L +ΔL) due to the recovering force of the resin, so as to make the distal end portion straight (Fig. 5 (B)).

The curvature at which the distal end of the catheter 10 is curved can be adjusted by controlling the degree at which the shape memory alloy wire members 16A and 16B recover their original shape, which is achieved by the control of the heating of the shape memory alloy wire members 16A and 16B by means of the control box 21.

In the catheter 10, since the distal end portion 12 is made of a material which is relatively soft and the shaft portion 11 is made of a material which is more rigid than that of the distal end portion 12, even if the deforming force of the shape memory alloy wire members 16A and 16B is small, the distal end of the catheter 10 can be curved adequately while the use of thin and short shape memory alloy wire members 16A and 16B is enabled.

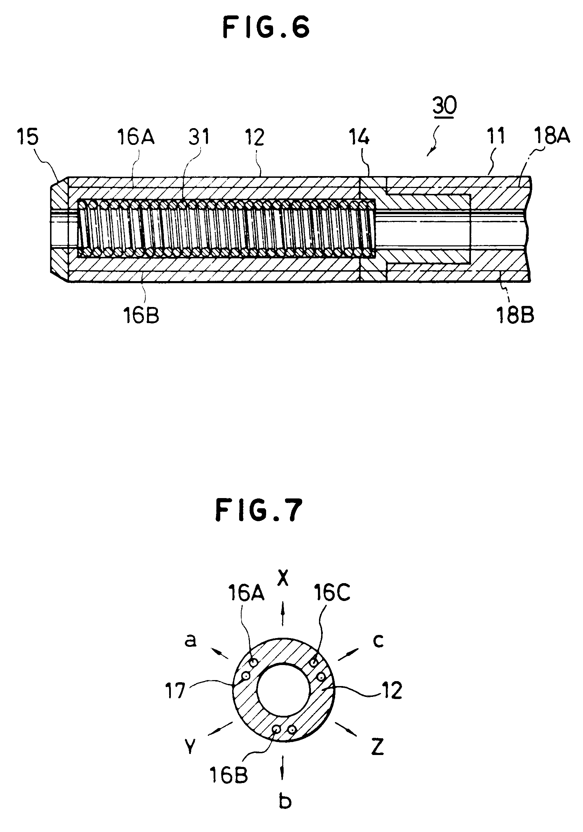

Fig. 6 is an enlarged cross-section of the essential parts of a second embodiment of the present invention.

The catheter 30 differs from the catheter 10 in that a cylindrical supporting member 31, made of a tight spring, is incorporated in the inner diameter portion of the distal end portion 12, i.e., inside of the shape memory alloy wire members 16A and 16B. The supporting member 31 is flexible in a direction in which it is bent, and is not compressed easily in its longitudinal direction so that it can impart to the distal end portion 12 a recovering force in the direction in which it is stretched.

In the catheter 30, then the shape memory alloy wire member 16A (or 16B) recovers its original shape and is contracted due to the heat generated when it is energized, the contracting force of the shape memory alloy wire member 16A is exerted only as the bending force on the distal end of the catheter 30 due to the presence of the cylindrical supporting member 31, generating no buckling based on the contracting force in the catheter 30. More specifically, changes in the shape of the shape memory alloy wire member 16A can be effectively utilized to bend the catheter 30.

When the distal end of the catheter 30 is to be returned to its straight state, the shape memory alloy wire member 16A is deenergized so as to make the distal end straight due to the stretching force of the supporting member 31.

A tube or the like which is capable of recovering its original shape in its axial direction may also be employed as the supporting member.

This embodiment may also be arranged such that three or more unidirectional shape memory alloy wire members are provided in the vicinity of the distal end of the catheter at positions which form a polygon within which the central axis of the catheter is disposed.

Fig. 7 shows a third embodiment of the present invention in which three unidirectional shape memory alloy wire members are disposed in the vicinity of the distal end of the catheter. Unidirectional shape memory alloy wire members 16A, 16B and 16C are disposed in the form of a triangle whose center is the central axis of the distal end portion 12. When it is desired to bend the distal end portion 12 in a direction indicated by the arrow a, only the alloy wire member 16A is energized When it is desired to bend the distal end portion 12 in a direction indicated by the arrow b or c, the alloy wire member 16B or 16C is similarly energized. When it is desired to bend in a direction indicated by the arrow X, both the alloy wire members 16A and 16C are energized so that the bending force in the direction a can be combined with the bending force in the direction C. Bending of the distal end portion in the direction indicated by Y or Z is achieved by the similar operation.

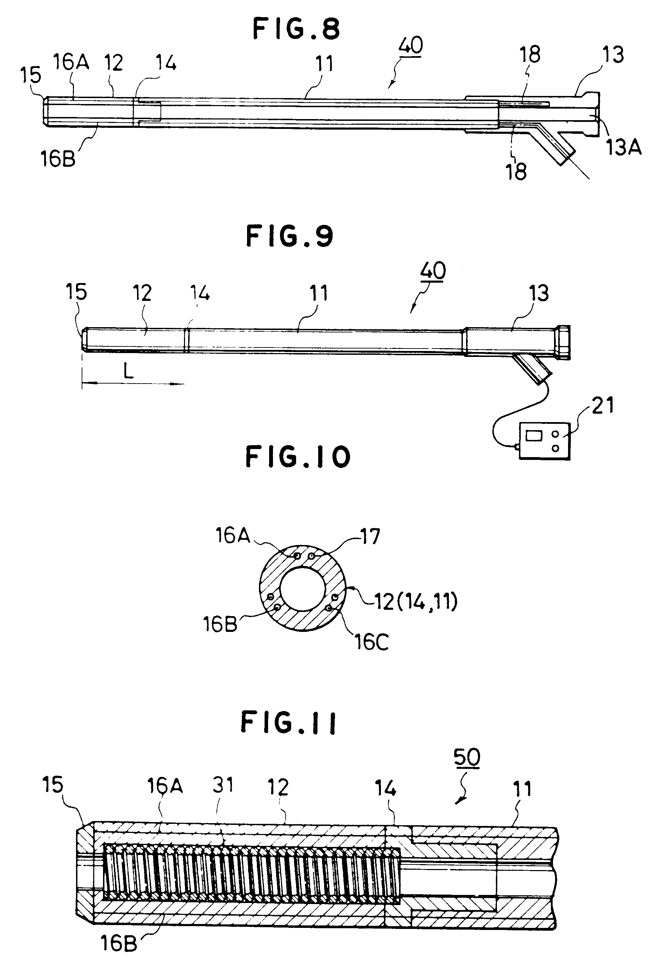

Figures 8 to 10 illustrate a fourth embodiment in which the catheter 40 differs from the catheter 10 in that it incorporates three shape memory alloy wire members 16A, 16B and 16C, like the catheter shown in Fig. 7, and in that these shape memory alloy wire members are provided along the overall length of the distal end portion 12 and the shaft portion 11. The wire members 16A through 16C are inserted in small holes 17 formed through the distal end portion 12, the fixed portion 14 and the shaft portion 11. They are turned in a U-shaped form at the distal end (at one end) thereof and are fixed to the proximal end portion of the shaft portion 11 (or to the end portion of the hub 13) and connected to the leads 18 at proximal end (the other end) thereof. The proximal end portion of the shaft portion 11 (or the end portion of the hub 13) which supports the shape memory alloy wire members 16A through 16C prevents the deforming force of the shape memory alloy wire members 16A through 16C from acting on the leads 18.

In the catheter 40, since the shape memory alloy wire members 16A through 16C are provided along the overall length of the distal end portion 12 and the shaft portion 11, the length of the shape memory alloy wire members 16A through 16C provided in the catheter 40 is long. In consequence, the amount of extension or contraction (the deformed length) of the shape memory alloy wire members 16A through 16C increases, allowing the distal end of the catheter 40 to be curved at a large curvature and enabling the use of thin and small shape memory alloy wire members 16A through 16C. At that time, since the distal end portion 12 of the catheter 40 is made of a material which is softer than that of the shaft portion 11, the large amount of extension or contraction of the shape memory alloy wire members 16A through 16C can be concentrated on the soft portion of the distal end of the catheter 40, allowing it to be curved more freely at a larger curvature and enabling the use of thin and small alloy wire members 16A through 16C.

In this catheter 40, the distal end portion 12 may also be formed by a resin which has a strong recovering force. In this way, the alloy wire member 16A, after being contracted by the energization, can be returned to its stretched state by means of the recovering force of the resin.

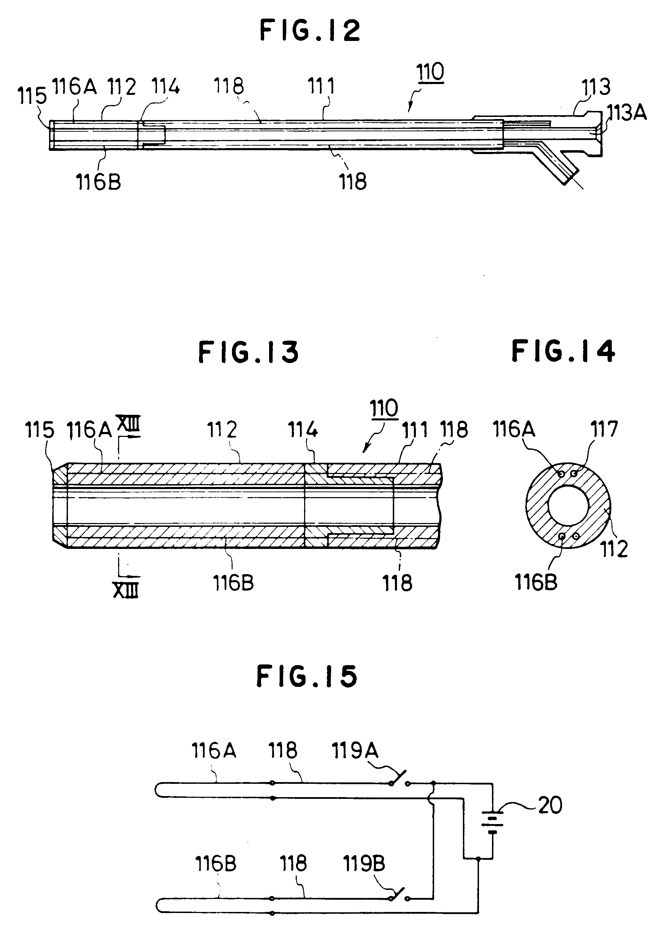

Fig. 11 is a cross-sectional view of the essential parts of a fifth embodiment of the catheter according to the present invention.

The catheter 50 differs from the catheter 40 in that the cylindrical supporting member 31, made of a tight spring, is incorporated in the inner diameter portion of the distal end portion 12, as in the case of the second embodiment shown in Fig. 6.

In this embodiment, the unidirectional shape memory alloy wire members may be provided in the wall of the catheter, on the inner wall surface or on the outer wall surface thereof.

The catheter of this invention may also be arranged such that it incorporates at least two shape memory alloy wire members which recover their original shape at a temperature lower than the set temperature and which is deformable at a temperature higher than the set temperature.

A Sixth embodiment is illustrated on figures 12 to 16

As illustrated on those figures the catheter 110 includes a relatively rigid, hollow shaft portion 111, a relatively flexible, hollow distal end portion 112, a hollow hub 113 which is bonded to the proximal end of the shaft portion 111, a relatively rigid, hollow fixed portion 114 which is provided between the shaft portion 111 and the distal end portion 112, and a relatively flexible, ring-shaped chip 115 which is bonded to the forward end of the distal end portion 112. The shaft portion 111 is made of, for example, polyvinyl chloride resin (which is non-rigid polyvinyl chloride resin containing less amount of plasticizing agent than that which forms the distal end portion 112). The distal end portion 112 is made of, for example, non-rigid vinyl chloride resin. The hub 113 is made of, for example, polycarbonate The fixed portion 114 and the soft chip 115 are made of, for example, polyvinyl chloride resin.

The catheter 110 further includes a pair of bidirectional shape memory alloy wire members 116A and 116B which are provided in the distal end portion 112. The two shape memory alloy wire members 116A and 116B are provided within the wall of the distal end portion 112 at positions which are symmetrical with respect to the central axis of the catheter 110 in such a manner that the longitudinal axis thereof is parallel to the axis of the catheter 110. The shape memory alloy wire members 116A and 116B are inserted into small holes 117 formed in the distal end portion 112. The forward end of each of the shame memory alloy wire members 116A and 116B is turned in a U-shaped form, and the proximal end thereof is supported by the fixed portion 114 and connected to leads 118. The fixed portion 114 prevents deformation of the shape memory alloy wire members 116A and 116B from being exerted on the shaft portion 111 and the leads 118.

The shape memory alloy wire members 116A and 116B are of the type which recovers its original different shapes at two different temperature which are substantially higher than the temperature of an organism (e.g., 60°C and 45 °C). More specifically, as the temperature chages from one of the two temperature to the other, the shape of the shape memory alloy wire members 116A and 116B reversibly changes in their longitudinal direction, e.g., the shape memory alloy wire members 116A and 116B recover their original shape and are contracted at the higher temperature which may be equal to or higher than 60°C, and they recover their original shape and are elongated at the lower temperature which may be 45°C.

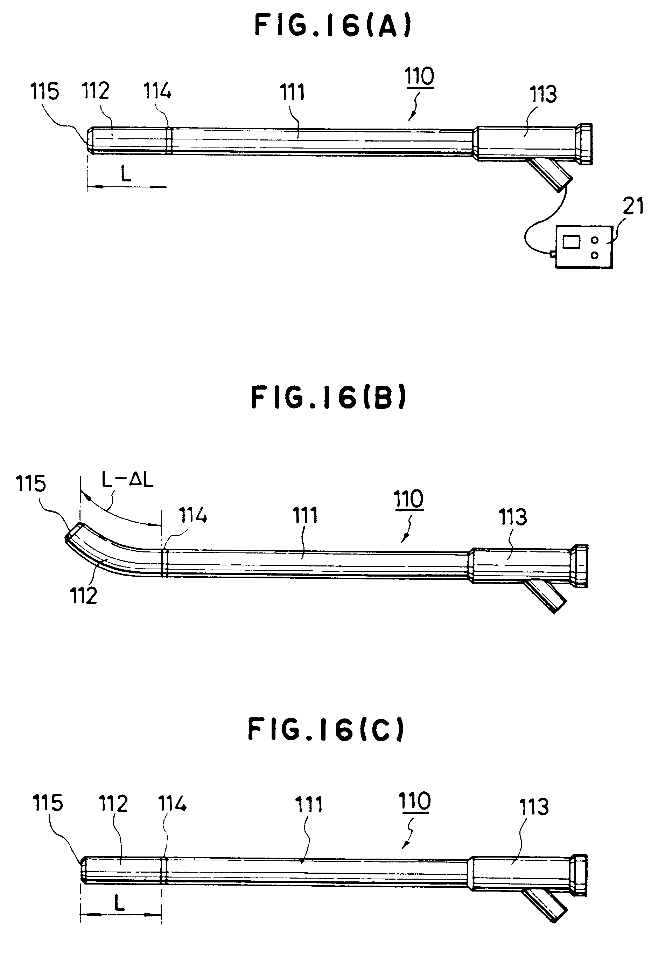

In this embodiment, the shape memory alloy wire members 116A and 116B are arranged such that they are elongated to a length which is substantially the same as the length L of the distal end portion 112 at the lower temperature and such that they are contracted and have a length which is shorter than the length of the distal end portion 112 by ΔL at the higher temperature (see Figs. 16 (A) through 16(C)). Also, the distal end portion 112 is arranged such that it is initially straight. at the lower temperature.

When the shape memory alloy wire members 116A and 116B are energized with an external power source 20 through the leads 118, heat is generated in the wire members due to an internal resistance thereof, thus heating them. Heating of the wire members 116A and 116B is controlled by the on/off of switches 119A and 119B. In Fig. 16(A), a reference numeral 21 denotes a control box which controls heating of the shape memory alloy wire members 116A and 116B.

Next, the operation of the sixth embodiment will be described.

When the catheter 110 is to be used, the operator forces the distal end portion 112 into an objective position within a blood vessel or the like by manually applying a pushing force to the proximal end portion of the shaft portion 111 while controlling the bending of the catheter distal end portion 112 which occurs when the shape memory alloy wire members 116A and 116B recover their original shape. Thereafter, the operator injects medicine or the like from an injection port 113A of the hub 113.

The shape memory alloy wire members 116A and 116B incorporated in the catheter 110 are initially in their original state which is obtained at the lower temperature which may be 45 °C, and have the length L which is substantially the same as that of the distal end portion 112. The initial shape of the distal end portion 112 is a straight shape (see Fig. 16(A)).

When one 116A of the shape memory alloy wire members is heated to the higher temperature which may be 60°C or above by means of the heat generated in the shape memory alloy wire member 116A due to its internal resistance when it is, for example, energized, the shape memory alloy wire member 116A recovers its original shape, i.e., it is contracted by ΔL. As a result, the initial shape of the distal end portion 112 chages and the distal end portion 112 is bent at a fixed curvature in a state in which the side of the distal end portion 112 where the shape memory alloy wire member 116A is provided is concave (see Fig. 16(B)).

Thereafter, the shape memory alloy wire member 116A may be deenergized so that it naturally cools down to the lower temperature. In consequence, the shape memory alloy wire member 116A recovers its original shape, i.e., it is elongated by ΔL to the length L which is substantially the same as that of the distal end portion 112, resulting in returning of the distal end portion 112 to its original straight shape (see Fig. 16(C)).

In this embodiment, since the two shape memory alloy wire members 116A and 116B are provided apart from each other in the distal end portion 112, the distal end portion 112 can be bend in two directions by selectively deforming an arbitrary one of the shape memory alloy wire members 116A and 116B.

Furthermore, the curvature at which the distal end portion 112 is bent can be adjusted by controlling the degree at which the shape memory alloy wire member 116A and 116B recover their original shape, which may be achieved by the control of the amount at which they are energized.

In the catheter 110, since the distal end portion 112 is made of a material which is relatively soft and the shaft portion 111 is made of a material which is more rigid than that of the distal end portion 112, even if the degree at which the shape memory alloy wire members 116A and 116B are deformed is small, the distal end of the catheter 110 can be curved adequately. Also, the use of thin and small shape memory alloy wire members 116A and 116B is enabled.

In this embodiment, three or more bidirectional shape memory alloy wire members may be provided in the vicinity of the distal end of the catheter at positions which forms a polygon within which the central axis of the catheter is disposed. At that time, the degree at which the shape memory alloy wire members are bent can be controlled and the curvature and direction at which the distal end portion of the catheter is bent can be thereby arbitrarily adjusted by arbitrarily selecting the shape memory alloy wire members to be energized and by changing the amount of heat generated therein.

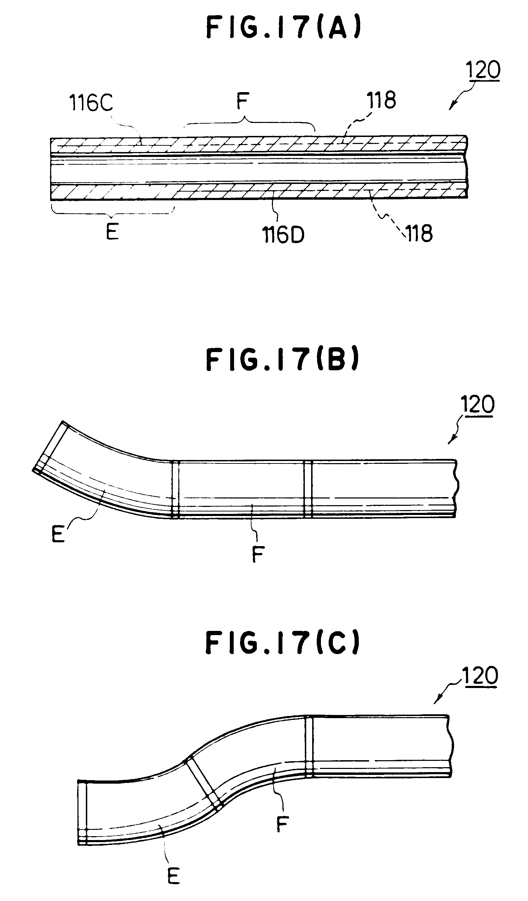

Fig. 17 shows a seventh embodiment of the present invention.

In this embodiment, a pair of bidirectional shape memory alloy wire members 116C and 116D are disposed in the manner shown in Fig. 17(A). More specifically, the bidirectional shape memory alloy wire member 116C is disposed at an E position in the vicinity of the forward end of the catheter while the shape memory alloy wire member 116D is disposed at an F portion which in on the side of the E portion closer to the proximal end of the catheter. When only the bidirectional shape memory alloy wire member 116C is energized and thereby heated, only the E portion in the vicinity of the forward end of the catheter can be bent in a state in which the side of the E portion where bidirectional shape memory alloy wire member 116C is provided is concave (see Fig. 17(B)). When the two bidirectional shape memory alloy wire members 116C and 116D are energized and heated, the F portion is bent in a state in which the side thereof in which the shape memory alloy wire member 116D is provided is concave while the E portion is bent in the above-described manner, thus achieving a complicated bending.

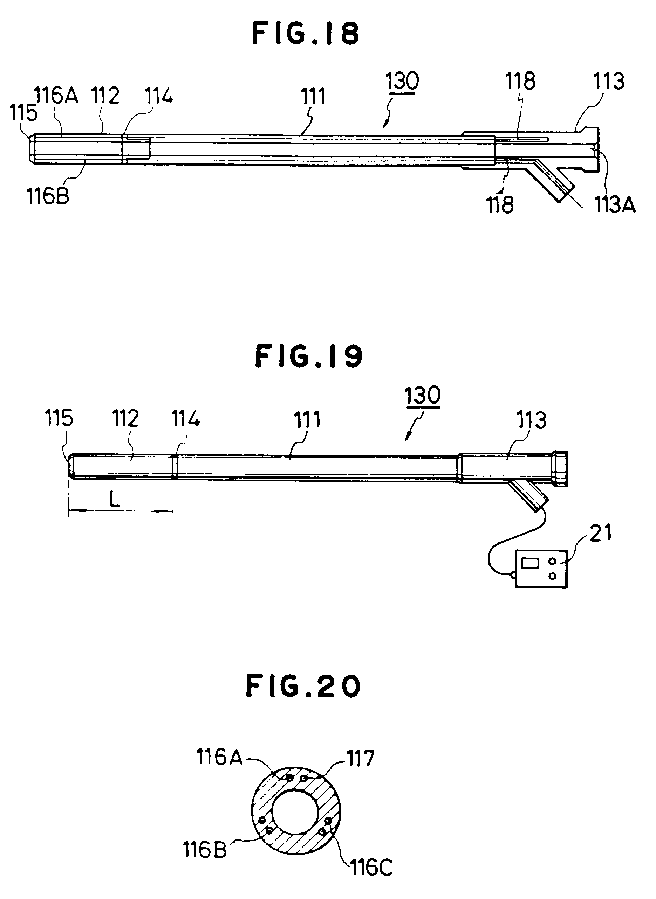

The Eighth embodiment of the invention is illustrated on figures 18 to 20.

As illustrated on those figures, the catheter 130 differs from the catheter 110 in that ① it incorporates three shape memory alloy wire members 116A, 116B and 116C and in that ② these shape memory alloy wire members are provided along the overall length of the disral end portion 112 and the shaft portion 111. The wire members 116A through 116C are inserted in small holes 117 formed in the distal end portion 112, the fixed portion 114 and the shaft portion 111. The forward end (one end) of each of the shape memory alloy wire members 116A through 116C is turned in a U-shaped form while the proximal end (the other end) thereof is fixed to the proximal end portion of the shaft portion 111 (or to the end portion of the hub 113) and connected to the leads 118. The proximal end portion of the shaft portion 111 (or the end portion of the hub 113) which supportes the shape memory alloy wire members 116A to 116C prevents deformation of the shape memory alloy wire members 116A through 116C from acting on the leads 118.

In the catheter 130, since the shape memory alloy wire members 116A through 116C are provided along the overall length of the distal end portion 112 and the shaft portion 111, the length of the shape memory alloy wire members 116A through 116C provided in the catheter 130 is long. In consequence, the degree of extension or contraction (the deformed length) of the shape memory alloy wire members 116A through 116C increases, and this allows the distal end of the catheter 130 to be curved at a large curvature. Also, the use of thin shape memory alloy wire members 116A through 116C is enabled. At that time, since the distal end portion 112 of the catheter 130 is made of a material which is softer than that of the shaft portion 111, the large degree of extension or contraction of the shape memory alloy wire members 116A through 116C can be concentrated on the soft portion which forms the forward end of the catheter 130, allowing it to be curved more freely at a larger curvature. Also, the use of thin and short alloy wire members 116A through 116C is enabled.

In this embodiment, the distal end portion of the catheter is initially straight. However, it may initially be in a state where it is bent at a fixed curvature, e.g., it may be bent in a J-shaped from. At that time, the distal end portion of the catheter may be of the type which undergoes deformation and is bent in a desired curvature or direction or is made straight when it is heated or cooled.

The catheter according to the present invention is inserted into a tubular organ of an organism, such as a blood vessel, an alimentary canal or an air tube. Insertion of the forward end of the catheter to an objective position in the tubular organ is achieved by the control of bending of the distal end portion, which occurs when the shape memory alloy wire member recovers its original shape, and the manual pushing operation applied to the proximal end portion of the shaft portion.

## Claims

1. A catheter (30) which is to be inserted into a tubular organ with at least two unidirectional shape memory alloy wire members (16A,16B), which recover their original shape at a temperature higher than a set temperature and which undergo deformation at a temperature lower than the set temperature provided at least in the distal end portion (12) of said catheter substantially parallel to an axis of said catheter and at positions which ensure that the force generated when at least one of said unidirectonal shape memory alloy wire members is heated to the temperature higher than the set temperature and thereby recovers its original shape exerts on the other unidirectional shape memory alloy wire member having a temperature lower than the set temperature a bending force, a cylindrical supporting member (31) being provided inside of said unidirectional shape memory alloy wire members, characterized in that said supporting member (31) is adapted to be easily flexed but not easily compressed in its longitudinal direction, and exerts a straightening recovering force to the shape memory alloy wire members (16A,16B) of the distal end portion when said supporting member (31) recovers its initial shape.

2. A catheter according to claim 1, wherein said distal end portion of said catheter is made of a material which is relatively flexible, and the portion of said catheter other than the distal end portion thereof is made of a material which is more rigid than that of said distal end portion.

3. A catheter according to either of claims 1 and 2, wherein said unidirectional shape memory alloy wire members are provided along both of said distal end portion and the other portion of said catheter, one end of each of said unidirectional shape memory alloy wire members being fixed to the distal end portion and the other end thereof being fixed to the other portion of the catheter.

4. A catheter according to either of claims 1 to 3, wherein said supporting member (31) is a tight spring.

5. A catheter according to either of claims 1 to 4, wherein two unidirectional shape memory alloy wire members (16A,16B) are disposed at positions which are opposed each other with respect to a central axis of said catheter.

6. A catheter according to either of claims 1 to 4, wherein three or more unidirectional shape memory alloy wire members (16A,16B,16C) are disposed at positions which form a polygon within which a central axis of said catheter is disposed.

7. A catheter according to either of claims 1 to 6, wherein said unidirectional shape memory alloy wire members (16A,16B,16C) are provided in a state in which they are elongated in their longitudinal direction.

8. A catheter according to either of claims 1 to 7, wherein said unidirectional shape memory alloy wire members (16A,16B,16C) are heated by means of the heat generated due to an internal resistance thereof when they are energized.

9. A catheter (110) which is to be inserted into a tubular organ having at least one flow passage which is formed therethrough along the overall length thereof, at least one bidirectional shape memory alloy wire member (116A,116B) being provided in a wall, on an inner wall surface or on an outer wall surface of said catheter in such a manner that it extends in an axial direction of said catheter, and in that the shape of a distal end portion (112) of said catheter reversibly changes by changing the temperature of the wire member, a cylindrical supporting member provided inside of said wall having the shape memory alloy wire member, characterized in that said supporting member is adapted to be easily flexed but not easily compressed and exerts a straightening recovering force to the shape memory alloy wire member of the distal end portion of the catheter when said cylindrical supporting member recovers its initial shape.

10. A catheter according to claim 9, wherein said distal end portion (112) of said catheter is made of a material which is relatively flexible, and the portion of said catheter other than said distal end portion (112) thereof is made of a material which is more rigid than that of said distal end portion.

11. A catheter according to either of claims 9 and 10, wherein said bidirectional shape memory alloy wire members (116A,116B) are provided along both of said distal end portion (112) and the portion of said catheter other than said distal end portion, one end of each of said bidirectional shape memory alloy wire members (116A,116B) being engaged to the distal end portion (112), and the other end thereof being engaged to the portion other than said distal end portion.

12. A catheter according to either of claims 9 to 11 , wherein said bidirectional shape memory alloy wire member (116A,116B) recovers its two original shapes at temperatures which are substantially higher than a body temperature.

13. A catheter according to either of claims 9 to 12, wherein an axis of said bidirectional shape memory alloy wire member (116A,116B) is substantially parallel to an axis of said catheter.

14. A catheter according to either of claims 9 to 13, wherein said bidirectional shape memory alloy wire member (116A,116B) changes its longitudinal shape as the temperature thereof changes.

15. A catheter according to either of claims 9 to 14, wherein two bidirectional shape memory alloy wire members (116A,116B) are disposed at positions which oppose each other with respect to a central axis of said catheter.

16. A catheter according to either of claims 9 to 14, wherein three or more bidirectional shape memory alloy wire members (116A,116B,116C) are disposed at positions which form a polygon within a central axis of said catheter is disposed.

17. A catheter according to either of claims 9 to 16, wherein said bidirectional shape memory alloy wire member is heated by means of the heat generated due to its internal resistance when said bidirectional shape memory alloy wire member is energized.

## Patentansprüche

1. Katheter (30), der in ein rohrförmiges Organ eingeführt werden soll, mit mindestens zwei Drahtelementen aus unidirektionaler Formgedächtnislegierung (16A, 16B), die bei einer höheren als einer festgesetzten Temperatur ihre ursprüngliche Form wiedergewinnen und die bei einer niedrigeren als der festgesetzten Temperatur zumindest im distalen Endabschnitt (12) des Katheters eine Verformung im wesentlichen parallel zu einer Achse des Katheters und an Positionen erfahren, die sicherstellen, daß die erzeugte Kraft, wenn zumindest eines der Drahtelemente aus unidirektionaler Formgedächtnislegierung auf die Temperatur höher als die festgesetzte Temperatur erwärmt wird und dadurch seine ursprüngliche Form wiedergewinnt, auf das andere Drahtelement aus unidirektionaler Formgedächtnislegierung, das eine niedrigere Temperatur als die festgesetzte Temperatur aufweist, eine Biegekraft ausübt, wobei ein zylindrisches Stützelement (31) innerhalb der Drahtelemente aus unidirektionaler Formgedächtnislegierung Vorgesehen ist, dadurch **gekennzeichnet,** daß das Stützelement (31) leicht biegbar aber in seiner Längsrichtung nicht leicht zusammendrückbar ist und auf die Drahtelemente aus Formgedächtnislegierung (16A, 16B) des distalen Endabschnittes eine geraderichtende Wiedergewinnungskraft ausübt, wenn das Stützelement (31) seine Ausgangsform wiedergewinnt.

2. Katheter nach Anspruch 1, bei dem der distale Endabschnitt des Katheters aus einem Material hergestellt ist, das verhältnismäßig flexibel ist, und der Abschnitt des Katheters verschieden von dem distalen Endabschnitt aus einem Material hergestellt ist, das steifer als das des distalen Endabschnittes ist.

3. Katheter nach einem der Ansprüche 1 und 2, bei dem die Drahtelemente aus unidirektionaler Formgedächtnislegierung entlang des distalen Endabschnittes und auch des anderen Abschnittes des Katheters vorgesehen sind, wobei ein Ende jedes der Drahtelemente aus unidirektionaler Formgedächtnislegierung an dem distalen Endabschnitt befestigt ist und wobei sein anderes Ende an dem anderen Abschnitt des Katheters befestigt ist.

4. Katheter nach einem der Ansprüche 1 bis 3, bei dem das Stützelement (31) eine feste Feder ist.

5. Katheter nach einem der Ansprüche 1 bis 4, bei dem zwei Drahtelemente aus unidirektionaler Formgedächtnislegierung (16A, 16B) an Positionen angeordnet sind, die in bezug auf eine zentrale Achse des Katheters einander gegenüberliegen.

6. Katheter nach einem der Ansprüche 1 bis 4, bei dem drei oder mehr Drahtelemente aus unidirektionaler Formmgedächtnislegierung (16A, 16B, 16C) an Positionen angeordnet sind, die ein Polygon bilden, innerhalb von dem eine zentrale Achse des Katheters angeordnet ist.

7. Katheter nach einem der Ansprüche 1 bis 6, bei dem die Drahtelemente aus unidirektionaler Formgedächtnislegierung (16A, 16B, 16C) in einem Zustand vorgesehen sind, in dem sie in ihrer Längsrichtung langgestreckt sind.

8. Katheter nach einem der Ansprüche 1 bis 7, bei dem die Drahtelemente aus unidirektionaler Formgedächtnislegierung (16A, 16B, 16C) mittels der Wärme erwärmt werden, die aufgrund eines Innenwiderstands von ihnen erzeugt wird, wenn sie stromführend sind.

9. Katheter (110), der in ein rohrförmiges Organ eingeführt werden soll, aufweisend mindestens einen Strömungsweg, der entlang seiner Gesamtlänge hindurch gebildet ist, wobei zumindest ein Drahtelement aus bidirektionaler Formgedächtnislegierung (116A, 116B) in einer Wand, an einer Innenwandfläche oder an einer Außenwandfläche des Katheters auf eine solche Weise vorgesehen ist, daß es sich in einer axialen Richtung des Katheters erstreckt, und daß sich die Form eines distalen Endabschnittes (112) des Katheters durch Änderung der Temperatur des Drahtelements reversibel ändert, wobei ein innerhalb der Wand vorgesehenes zylindrisches Stützteil, das Drahtelement mit Formgedächtnislegierung aufweist, dadurch **gekennzeichnet,** daß das Stützelement leicht biegbar, aber nicht leicht zusammendrückbar ist und auf das Drahtelement aus Formgedächtnislegierung des distalen Endabschnittes des Katheters eine geraderichtende Wiedergewinnungskraft ausübt, wenn das zylindrische Stützelement (31) seine Ausgangsform wiedergewinnt.

10. Katheter nach Anspruch 9, bei dem der distale Endabschnitt (112) des Katheters aus einem Material hergestellt ist, das verhältnismäßig flexibel ist, und der Abschnitt des Katheters verschieden von dessen distalem Endabschnitt (112) aus einem Material hergestellt ist, das steifer als das des distalen Endabschnittes ist.

11. Katheter nach einem der Ansprüche 9 und 10, bei dem die Drahtelemente aus bidirektionaler Formgedächtnislegierung (116A, 116B) entlang des distalen Endabschnittes (112) und auch des Abschnitts des Katheters verschieden von dem distalen Endabschnitt vorgesehen sind, wobei sich ein Ende jedes der Drahtelemente aus bidirektionaler Formgedächtnislegierung (116A, 116B) mit dem distalen Endabschnitt (112) im Eingriff befindet und wobei sich ihr anderes Ende mit dem Abschnitt verschieden von dem distalen Endabschnitt im Eingriff befindet.

12. Katheter nach einem der Ansprüche 9 bis 11, bei dem das Drahtelement aus bidirektionaler Formgedächtnislegierung (116A, 116B) seine zwei ursprünglichen Formen bei Temperaturen wiedergewinnt, die wesentlich höher als eine Körpertemperatur sind.

13. Katheter nach einem der Ansprüche 9 bis 12, bei dem eine Achse des Drahtelements aus bidirektionaler Formgedächtnislegierung (116A, 116B) im wesentlichen parallel zu einer Achse des Katheters ist.

14. Katheter nach einem der Ansprüche 9 bis 13, bei dem das Drahtelement aus bidirektionaler Formgedächtnislegierung (116A, 116B) seine Form in Längsrichtung verändert, wenn sich seine Temperatur ändert.

15. Katheter nach einem der Ansprüche 9 bis 14, bei dem zwei Drahtelemente aus bidirektionaler Formgedächtnislegierung (116A, 116B) an Positionen angeordnet sind, die in bezug auf eine zentrale Achse des Katheters einander gegenüberliegen.

16. Katheter nach einem der Ansprüche 9 bis 14, bei dem drei oder mehr Drahtelemente aus bidirektionaler Formgedächtnislegierung (116A, 116B, 116C) an Positionen angeordnet sind, die ein Polygon bilden, innerhalb von dem eine zentralen Achse des Katheters angeordnet ist.

17. Katheter nach einem der Ansprüche 9 bis 16, bei dem das Drahtelement aus bidirektionaler Formgedächtnislegierung mittels der Wärme erwärmt wird, die aufgrund seines Innenwiderstands erzeugt wird, wenn das Drahtelement aus bidirektionaler Formgedächtnislegierung stromführend ist.

## Revendications

1. Cathéter (30) destiné à être introduit dans un organe tubulaire, avec au moins deux éléments de fils métalliques (16A, 16B)
en alliage à mémoire de forme unidirectionnelle, qui retrouvent leur forme de départ à une température supérieure à une température donnée et qui subissent une déformation à une température inférieure à la température donnée, placés dans la partie (12) d'extrémité distale au moins dudit cathéter en étant sensiblement parallèles à l'axe dudit cathéter et en des positions qui garantissent que la force, produite quand l'un au moins desdits éléments de fils métalliques en alliage à mémoire de forme unidirectionnelle est chauffé à la température supérieure à la température donnée et retrouve de ce fait sa forme de départ, exerce sur l'autre fil métallique en alliage à mémoire de forme, ayant une température inférieure à la température donnée, une force de flexion, un élément de support (31) cylindrique, étant placé à l'intérieur desdits éléments de fils métalliques en alliage à mémoire de forme unidirectionnelle, caractérisé en ce que ledit élément de support (31) peut être facilement fléchi mais ne peut pas être facilement comprimé dans sa direction longitudinale et exerce une force de rappel sur les éléments de fils métalliques (16A, 16B) en alliage à mémoire de forme de la partie d'extrémité distale quand ledit élément de support (31) retrouve sa forme initiale.

2. Cathéter selon la revendication 1, dans lequel ladite partie d'extrémité distale dudit cathéter est faite d'un matériau qui est relativement souple et la partie dudit cathéter autre que sa partie d'extrémité distale est faite d'un matériau qui est plus rigide que celui de ladite partie d'extrémité distale.

3. Cathéter selon la revendication 1 ou 2, dans lequel lesdits éléments de fils métalliques en alliage à mémoire de forme unidirectionnelle sont placés à la fois le long de ladite partie d'extrémité distale et de l'autre partie dudit cathéter, une extrémité de chacun desdits fils métalliques en alliage à mémoire de forme unidirectionnelle étant fixée à la partie d'extrémité distale et son autre extrémité étant fixée à l'autre partie du cathéter.

4. Cathéter selon l'une des revendications 1 à 3, dans lequel ledit élément de support (31) est un ressort à spires jointives.

5. Cathéter selon l'une des revendications 1 à 4, dans lequel deux éléments de fils métalliques (16A, 16B) en aliage à mémoire de forme unidirectionnelle sont placés en des positions qui sont opposées l'une de l'autre par rapport à l'axe central dudit cathéter.

6. Cathéter selon l'une des revendications 1 à 4, dans lequel trois éléments de fils métalliques (16A, 16B, 16C) en alliage à mémoire de forme unidirectionnelle, ou plus, sont placés en des positions qui forment un polygone à l'intérieur duquel est placé l'axe central dudit cathéter.

7. Cathéter selon l'une quelconque des revendications 1 à 6, dans lequel lesdits fils métalliques (16A, 16B, 16C) en alliage à mémoire de forme unidirectionnelle sont placés de telle manière qu'ils sont allongés dans leur direction longitudinale.

8. Cathéter selon l'une quelconque des revendications 1 à 7, dans lequel lesdits fils métalliques (16A, 16B, 16C) en alliage à mémoire de forme unidirectionnelle sont chauffés au moyen de la chaleur produite par leur résistance interne lorsqu'ils sont alimentés en énergie.

9. Cathéter (110) destiné à être introduit dans un organe tubulaire, ayant au moins un passage d'écoulement qui le traverse sur toute sa longueur, au moins un fil métallique (116A, 116B) en alliage à mémoire de forme bidirectionnelle étant placé dans une paroi, sur une surface de paroi interne ou sur une surface de paroi externe dudit cathéter de manière à s'étendre dans la direction axiale dudit cathéter, et à ce que la forme de la partie d'extrémité distale (112) dudit cathéter change de façon réversible parmodification de la température du fil métallique, un élément de support cylindrique placé à l'intérieur de ladite paroi comprenant le fil métallique en alliage à mémoire de forme, caractérisé en ce que ledit élément de support peut être facilement fléchi mais pas facilement comprimé et exerce une force de rappel sur le fil métallique en alliage à mémoire de forme de la partie d'extrémité distale du cathéter quand ledit élément de support cylindrique retrouve sa forme initiale.

10. Cathéter selon la revendication 9, dans lequel ladite partie (112) d'extrémité distale dudit cathéter est faite d'un matériau qui est relativement souple et la partie dudit cathéter autre que ladite partie (112) d'extrémité distale est faite d'un matériau qui est plus rigide que celui de ladite partie d'extrémité distale.

11. Cathéter selon l'une des revendications 9 ou 10, dans lequel lesdits éléments de fils métalliques (116A, 116B) en alliage à mémoire de forme bidirectionnelle sont placés à la fois le long de ladite partie (112) d'extrémité distale et de la partie dudit cathéter autre que ladite partie d'extrémité distale, une extrémité de chacun desdits fils métalliques (116A, 116B) en alliage à mémoire de forme bidirectionnelle étant fixée sur la partie (112) d'extrémité distale et son autre extrémité étant fixée sur la partie autre que ladite partie d'extrémité distale.

12. Cathéter selon l'une quelconque des revendications 9 à 11, dans lequel ledit fil métallique (116A, 116B) en alliage à mémoire de forme bidirectionnelle retrouve ses deux formes d'origine à des températures qui sont sensiblement plus élevées que la température du corps.

13. Cathéter selon l'une quelconque des revendications 9 à 12, dans lequel l'axe dudit fil métallique (116A, 116B) en alliage à mémoire de forme bidirectionnelle est sensiblement parallèle à l'axe dudit cathéter.

14. Cathéter selon l'une quelconque des revendications 9 à 13, dans lequel ledit fil métallique (116A, 116B) en alliage à mémoire de forme bidirectionnelle change de forme longitudinale lorsque sa température change.

15. Cathéter selon l'une quelconque des revendications 9 à 14, dans lequel deux fils métalliques (116A, 116B) en alliage à mémoire de forme bidirectionnelle sont placés en des positions qui sont opposées l'une de l'autre par rapport à l'axe central dudit cathéter.

16. Cathéter selon l'une quelconque des revendications 9 à 14, dans lequel trois fils métalliques (116A, 116B, 116C) en alliage à mémoire de forme bidirectionnelle, ou plus, sont placés en des positions qui forment un polygone à l'intérieur duquel est placé l'axe central dudit cathéter.

17. Cathéter selon l'une quelconque des revendications 9 à 16, dans lequel ledit fil métallique en alliage à mémoire de forme bidirectionelle est chauffé au moyen de la chaleur produite par sa résistance interne quand ledit fil métallique en alliage à mémoire de forme bidirectionnelle est alimenté en énergie.
